# EUROPEAN PATENT SPECIFICATION

(11) **EP 4 184 129 B1**
(45) Date of publication and mention of the grant of the patent: **10.09.2025**
(21) Application number: 22208598.7
(22) Date of filing: 21.11.2022
(51) Int. Cl.: G01F 15/00, G01M 3/26, G01F 15/063

(54) **LOCAL SAFETY FEATURES FOR GAS METERS WITH LOCAL GAS DETECTOR PAIRING AND MULTI STACK SUPPORT**
LOKALE SICHERHEITSMERKMALE FÜR GASZÄHLER MIT LOKALER GASDETEKTORPAARUNG UND MULTISTACK-UNTERSTÜTZUNG
CARACTÉRISTIQUES DE SÉCURITÉ LOCALES POUR COMPTEURS À GAZ AVEC APPARIEMENT LOCAL DE DÉTECTEURS DE GAZ ET SUPPORT À EMPILEMENT MULTIPLE

(30) Priority: 23.11.2021 US 202117456262
(43) Date of publication of application: 24.05.2023
(73) Proprietor: Honeywell International Inc., Charlotte, NC 28202 (US)
(72) Inventor: SAMUDRALA, Prasad, Charlotte 28202 (US); MATHEW, Mithun, Charlotte 28202 (US); BEZAWADA, Murali Krishna, Charlotte 28202 (US); TEMME, Guido, Charlotte 28202 (US)
(74) Representative: Houghton, Mark Phillip

(56) References cited:
- CN-A- 108 106 684
- JP-A- 2011 197 748
- JP-A- 2012 018 502
- JP-U- H0 596 748
- US-A1- 2020 042 021

## Description

### TECHNICAL FIELD

The present disclosure generally relates to safety features for gas meters in response to detections of gas leaks by gas detectors in the same system.

### BACKGROUND

Gas meters in a system are typically battery-operated devices, they can be in sleep mode at various intervals during the course of a day or week to preserve the battery energy to keep meter running for many years. The gas meter also has an integrated shut off valve to shut off the flow of gas in various conditions. One such condition is when a gas leak detector detects a gas leak, it reports the gas meter about the gas leak level and meter shuts off the gas flow While the gas meter is in sleep mode, gas leaks can occur within the gas system. Moreover, when the gas leaks occur, the gas meter being in sleep mode may not be notified of the detected gas leak.

Another consideration is that a gas meter should be able to communicate with other entities such as a head end system and gas detector. When the gas meter is in sleep mode, it is unable to communicate with either a gas detector and a head end system. Moreover, a gas leak can continue while the gas meter is in sleep mode.

As such, a need exists for the gas meter to be alerted when the gas meter is in sleep mode. The gas meter should be able to receive notification when there is a potential leak so that that potential leak can be prevented.

In addition, a need also exists for the gas meter to be able to communicate its data included gas level received from gas detector and its gas valve status with a head end system. The gas meter needs to be alerted to a potential gas leak, and communicate with both a gas detector and head end system to help prevent the gas leak from occurring.

Both gas meter and gas detector are battery operated wireless devices running in very low power mode of operation, whereas meter continue to measure the gas flow volume and detector continue to monitor the gas leak levels. Beyond that they are always sleeping devices. Both gas meter and gas detector communicate wirelessly with always sleeping devices. Both gas meter and gas detector communicate wirelessly with each other and also gas meter communicates with head end system over wireless network.

Examples of currently used systems can be found in the following documents:
JP2012018502A, which discloses a gas cutoff device that includes a plurality of detection thresholds, and outputs a detected gas value as a signal corresponding to a stepwise level based on the plurality of thresholds. An alarm device comprising a detection means, an alarm transmission means for transmitting an output signal of the gas detection means to a gas meter, a cutoff means for supplying or shutting off the gas, and a gas meter for receiving the output signal transmitted from the alarm device. gas meter control means for processing an output signal transmitted from the gas meter receiving means; gas flow rate detection means for detecting the flow rate of gas flowing through the gas meter; and a gas meter equipped with a gas leak detection means for detecting a leak, and when the gas leak detection means detects a gas leak in the gas pipe, the gas is shut off based on a low detection threshold among the output signals.
CN108106684A, which discloses a smart gas meter that prevents a small amount of gas leakage, including a gas monitoring and alarm module, that includes at least one gas detector, a control module, a solenoid valve module, remote communication module, sound and light alarm module and power module, gas detector, solenoid valve module. The signal input by the sensor is used to judge and process the leakage, and respectively control the solenoid valve module to shut off the gas intake, the remote communication module and the sound and light alarm module to issue an alarm; and the smart gas meter also includes a gas meter, which is connected to the gas monitoring and alarm module. The control module also includes a micro-leakage judging module, which monitors the measurement value of the gas meter, judges and handles micro-leakage and abnormal gas consumption in combination with the gas detector signal, and controls the solenoid valve module to shut off the gas intake and the remote communication module respectively. and the sound and light alarm module to issue prompts or alarms.

### SUMMARY

The following summary is provided to facilitate an understanding of some of the features of the disclosed embodiments and is not intended to be a full description. A full appreciation of the various aspects of the embodiments disclosed herein can be gained by taking the specification, claims, drawings, and abstract as a whole.

The aforementioned aspects and other objectives can now be achieved as described herein.

According to the invention, a system includes a gas detector configured to detect a gas leak and provide a notification to a gas meter regarding a gas level of the gas leak, wherein the gas meter includes a gas valve configured to shut off or switch on the gas flow. The system also includes the gas meter configured to receive the notification about the gas leak from the gas detector. The gas meter compares the gas level detected by the gas detector with a threshold level and determines if the detected gas level is greater than the threshold level to determine whether to turn off a gas valve due to the detected gas level. The system also includes a head end system configured to receive notification of the detected gas level and status of the gas valve from the gas meter.

According to the invention, the gas meter is in sleep mode when the gas detector detects the gas level in relation to the gas leak.

According to the invention, the gas detector sends a wakeup signal to the gas meter when a gas leak is detected. The gas meter detects the wakeup signal, checks for authenticity of the detector which sent the wakeup signal and receives the gas level information.

The gas meter closes the gas valve when the received gas level is greater than the threshold level.

In an embodiment, a system includes a gas detector configured at a first position. The gas detector is configured to detect a gas leak of a gas meter configured at a second position. The gas detector sends a wakeup signal to the gas meter due to the gas meter being in sleep mode. The system also includes the gas meter configured to receive the wakeup signal from the gas detector. The gas meter compares a gas detector level associated with the gas leak, to a threshold level and determines whether to close a gas valve associated with the gas meter, and report its data to a data head end system. The system also includes the head end system configured to receive the data from the gas meter. The data includes the determination as to whether the gas meter turned off the gas valve due to the comparison of the gas level associated with the gas leak to the threshold level.

The gas meter determines to close the gas valve due to the comparison of the gas level to the threshold level.

The gas meter reports the data regarding the gas level and gas valve status to the head end system in periodic levels.

According to the invention, the gas meter receives the wakeup signal in one frequency and switches to a different frequency in response to the wake up signal from the gas detector to exchange data with the gas detector.

In an embodiment, a method includes configuring a gas detector to detect a gas leak and provide a notification to a gas meter regarding a gas level of the gas leak. The method also includes positioning the gas meter to receive the notification about the gas leak from the gas detector. Further the gas meter compares the gas level detected by the gas detector with a threshold level and determines if the detected gas level is greater than the threshold level to determine whether to turn off a gas valve due to the detected gas level. The method also includes configuring a head end system to receive notification of the detected gas level and status of the gas valve from the gas meter.

The gas meter communicates to the head end system whether the gas valve was turned off due to the detected gas level.

The gas detector sends a wakeup signal to the gas detector on a RF wake-up channel operating at a frequency of 451.4 mega-Hertz.

The gas meter wakes up when it sees wakeup signal on the RF wake-up channel, switches to RF data channel operating at 915MHz frequency band to receive the gas level information from gas detector & informs the gas detector that it received the gas leak level information.

The gas meter on receiving the gas level from gas detector, checks the gas level with the configured threshold to close the valve. If the gas level is greater than the configured threshold, the gas meter switches off the valve and reports the gas level and the valve status to head end system immediately, with different or the same communication protocol, possibly even in the same RF data channel frequency bands.

### BRIEF DESCRIPTION OF THE FIGURES

The accompanying figures, in which like reference numerals refer to identical or functionally similar elements throughout the separate views and which are incorporated inf and form a part of the specification, further illustrate the present invention and, together with the detailed description of the invention, serve to explain the principles of the present invention.
**FIG. 1** illustrates a system diagram in accordance with an embodiment of the invention;
**FIG. 2** illustrates schematic diagram in accordance with the invention;
**FIG. 3** illustrates frequency diagrams in accordance with an embodiment of the invention; and
**FIG. 4** illustrates a block diagram in accordance with an embodiment of the invention.
**FIGS. 5(A)****-(B)** illustrates flowcharts in accordance with embodiments of the invention.
**FIG. 6** depicts a flow chart in accordance with an embodiment of the invention.

Unless otherwise indicated illustrations in the figures are not necessarily drawn to scale.

### DETAILED DESCRIPTION OF SOME EMBODIMENTS

### Background and Context

The particular values and configurations discussed in these non-limiting examples can be varied and are cited merely to illustrate one or more embodiments and are not intended to limit the scope thereof.

Subject matter will now be described more fully herein after with reference to the accompanying drawings, which form a part hereof, and which show, by way of illustration, specific example embodiments. Subject matter may, however, be embodied in a variety of different form and, therefore, covered or claimed subject matter is intended to be construed as not being limited to any example embodiments set forth herein, example embodiments are provided merely to be illustrative. Likewise, a reasonably broad scope for claimed or covered subject matter is intended. Among other issues, subject matter may be embodied as methods, devices, components, or systems. The followed detailed description is, therefore, not intended to be interpreted in a limiting sense.

Throughout the specification and claims, terms may have nuanced meanings suggested or implied in context beyond an explicitly stated meaning. Likewise, phrases such as "in one embodiment" or "in an example embodiment" and variations thereof as utilized herein may not necessarily refer to the same embodiment and the phrase "in another embodiment" or "in another example embodiment" and variations thereof as utilized herein may or may not necessarily refer to a different embodiment. It is intended, for example, that claimed subject matter include combinations of example embodiments in whole or in part.

In general, terminology may be understood, at least in part, from usage in context. For example, terms such as "and," "or," or "and/or" as used herein may include a variety of meanings that may depend, at least in part, upon the context in which such terms are used. Generally, "or" if used to associate a list, such as A, B, or C, is intended to mean A, B, and C, here used in the inclusive sense, as well as A, B, or C, here used in the exclusive sense. In addition, the term "one or more" as used herein, depending at least in part upon context, may be used to describe any feature, structure, or characteristic in a singular sense or may be used to describe combinations of features, structures, or characteristics in a plural sense. Similarly, terms such as a "a," "an," or "the", again, may be understood to convey a singular usage or to convey a plural usage, depending at least in part upon context. In addition, the term "based on" may be understood as not necessarily intended to convey an exclusive set of factors and may, instead, allow for existence of additional factors not necessarily expressly described, again, depending at least in part on context.

One having ordinary skill in the relevant art will readily recognize the subject matter disclosed herein can be practiced without one or more of the specific details or with other methods. In other instances, well-known structures or operations are not shown in detail to avoid obscuring certain aspects. This disclosure is not limited by the illustrated ordering of acts or events, as some acts may occur in different orders and/or concurrently with other acts or events. Furthermore, not all illustrated acts or events are required to implement a methodology in accordance with the embodiments disclosed herein.

Unless defined otherwise, all technical and scientific terms used herein have the same meanings as commonly understood by one of ordinary skill in the art to which the disclosed embodiments belong. Preferred methods, techniques, devices, and materials are described, although any methods, techniques, devices, or materials similar or equivalent to those described herein may be used in the practice or testing of the present invention.

References "an embodiment," "example embodiment," "various embodiments," "some embodiments," etc., may indicate that the embodiment(s) so described may include a particular feature, structure, or characteristic, but not every possible embodiment necessarily includes that particular feature, structure, or characteristic.

Headings provided are for convenience and are not to be taken as limiting the present disclosure in any way.

Each term utilized herein is to be given its broadest interpretation given the context in which that term is utilized.

### Terminology

The following paragraphs provide context for terms found in the present disclosure (including the claims):
The transitional term "comprising", which is synonymous with "including," "containing," or "characterized by," is inclusive or open-ended and does not exclude additional, unrecited elements or method steps. See, e.g., *Mars Inc. v. H.J. Heinz Co.,* 377 F.3d 1369, 1376, 71 USPQ2d 1837, 1843 (Fed. Cir. 2004) ("[L]ike the term 'comprising,' the terms 'containing' and 'mixture' are open-ended."). "Configured to" or "operable for" is used to connote structure by indicating that the mechanisms/units/components include structure that performs the task or tasks during operation. "Configured to" may include adapting a manufacturing process to fabricate components that are adapted to implement or perform one or more tasks.

"Based On." As used herein, this term is used to describe factors that affect a determination without otherwise precluding other or additional factors that may affect that determination. More particularly, such a determination may be solely "based on" those factors or based, at least in part, on those factors.

All terms of example language (e.g., including, without limitation, "such as", "like", "for example", "for instance", "similar to", etc.) are not exclusive of other examples and therefore mean "by way of example, and not limitation... ".

A description of an embodiment having components in communication with each other does not infer that all enumerated components are needed.

The flowchart and block diagrams in the figures illustrate the architecture, functionality, and operation of possible implementations of systems and methods according to various embodiments. Functions noted in the block may occur out of the order noted in the figures. For example, two blocks shown in succession may, in fact, be executed substantially concurrently, or the blocks may sometimes be executed in the reverse order, depending upon the functionality involved.

Further, any sequence of steps that may be described does not necessarily indicate a condition that the steps be performed in that order. Some steps may be performed simultaneously.

The functionality and/or the features of a particular component may be alternatively embodied by one or more other devices that are not explicitly described as having such functionality/features.

More specifically, as will be appreciated by one skilled in the art, aspects of the present invention may be embodied as a system and/or method. Furthermore, aspects of the present invention may take the form of a plurality of systems to enable gas meter to perform self-checking to determine its overall functioning without requiring a meter operator.

### Introduction

Embodiments of the present invention include wireless gas meter being positioned in the same system as a wireless gas detector and head end system. The gas meter can be in sleep mode at various intervals in the system. The gas detector can be configured with a sensor (methane, propane, environmental) to monitor the gas level of the gas meter. As such, the gas detector can monitor the gas level of the gas meter while the gas meter is in sleep mode.

The gas detector, or sensor within the gas detector, can detect a gas level of the gas meter that can be a suspected gas leak. Since the gas meter can be in sleep mode, the gas detector can send a wakeup notification to the gas meter at a signal of 451.4 Megahertz (Mhz). The gas meter can receive the signal and switch to a communication frequency of 915 Mhz to communicate with both the head end system and the gas detector. The gas meter can also send acknowledgement of the gas level received to the gas detector.

The gas meter will compare the detected gas level to a threshold level to determine if a gas leak exists. As such, if the detected gas level is greater than the threshold level, the gas meter will determine that a gas leak exists and close a gas valve within the gas meter to prevent any further damage due to the gas leak. In the alternative, the gas meter can decide to leave the gas valve on if the detected gas level is less than the threshold level.

The gas meter will also communicate its data with the head end system. The gas meter will inform the head end system of the detected gas level and whether or not the detected gas level was a gas leak. The gas meter will also communicate to the head end system its valve status, and whether or not the gas valve was turned off or not.

The gas meter can communicate with the head end system and the gas detector at a communication frequency of 915 Mhz. The gas meter can periodically communicate with both the gas detector and the head end system regarding its gas level and its valve status. While in sleep mode, the gas meter will always be alerted by the gas detector when the gas detector has detected a gas level that can potentially be a gas leak. Moreover, the gas meter will always be alerted to determine whether or not to turn off the gas valve due to the detected gas level.

### System structure

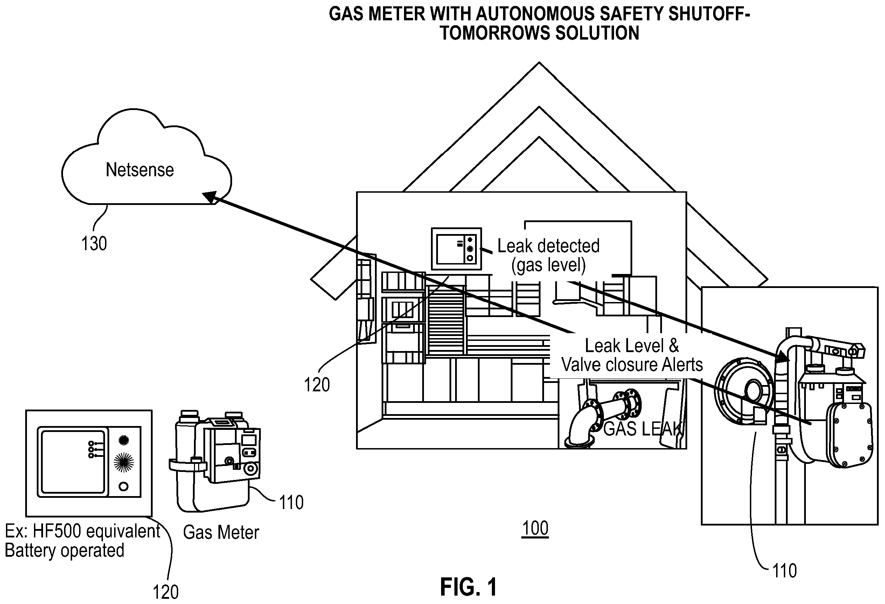

**FIG. 1** illustrates gas detection system **100.** The gas detection system **100** includes a gas meter **110** and a gas detector **120,** and a netsense or head end system **130.** During the course of a day or interval, the gas meter **110** can have gas levels that can amount to a gas leak. The gas detector **120** will consistently monitor the gas levels of the gas meter **110,** and also periodically exchange data with the gas meter **110.** When the gas detector **120** determines that the gas meter **110** has a gas level that amounts to a gas leak, the gas detector **120** will send a wake-up signal to the gas meter **110.** The gas detector **110** will send a wakeup signal in the frequency range of 451.4 Mhz. The gas detector **100** can send the wakeup signal to the gas meter **110** as the gas meter **110** is in sleep mode.

Referring to **FIG. 1****,** after the gas meter **110** has received the wakeup signal/notification from the gas detector, the gas meter can determine whether the detected gas level is an actual gas leak. The gas meter **110** can compare the detected gas level with a threshold level. If the gas level is less than the threshold level, the gas meter can determine that a gas leak is not present. In contrast, if the detected gas level is greater than the threshold level, the gas meter can determine that a gas leak is present, and decide to turn off a valve within the gas meter in response to the gas leak.

In **FIG. 1****,** after the gas meter has determined whether the detected gas level is greater than or less than the threshold level, the gas meter **110** can report its data to the head end system **130.** The gas meter **110** will inform the head end system **130** whether a gas leak exists. Further, the gas meter **110** can inform the head end system of the status of the gas valve, and whether the gas valve has been turned off or has remained on. Moreover, the gas meter **110** can periodically report its data to the head end system in relation to the gas level and to the valve status. The gas meter **110** can exchange data periodically with both the gas detector **120** and the head end system **130.**

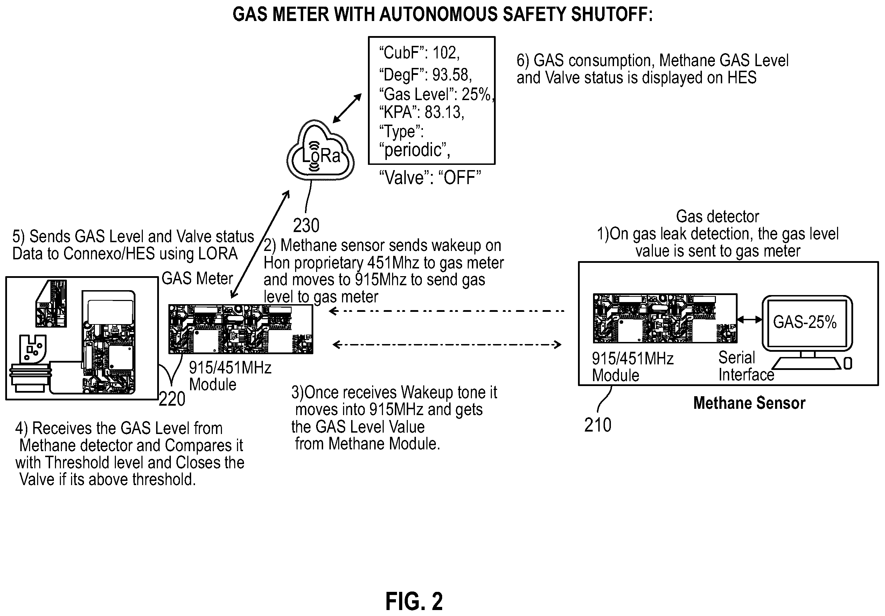

Referring to **FIG. 2****,** a schematic diagram of the gas detection system is illustrated. A methane sensor **210** found within a gas detector is shown. In other embodiments, a propane sensor or environmental sensor can be configured within the gas detector. The internal diagram of a gas meter **220** is shown as well. In addition, a head end system **230** LoRa is shown as well.

In **FIG. 2****,** the methane sensor **210** can monitor the gas levels of the gas meter **220.** The gas meter **220** can be in sleep mode as the methane sensor **210** is monitoring the gas levels of the gas meter **220.** The methane sensor **210** can send a wakeup notification or signal to the gas meter **220** when the methane sensor **210** senses a gas level that can be a gas leak. The wakeup notification will come at a signal of 451.4 Mhz and then gas meter switches to 915Mhz to receive the gas level information from detector. The gas meter **220** can be alerted to the detected gas level, and then compare the detected gas level to the threshold level to determine if the detected gas level is less than or equal to the threshold level. The gas meter **220** can then determine whether a gas leak exists or not based on the comparison of the detected gas level with the threshold level. Further, the gas meter **220** determines whether to turn off a gas valve based on whether a gas leak is present. The gas meter **220** turns off the gas valve when the gas leak is present.

With respect to **FIG. 2****,** the gas meter reports its data to the head end system **230.** The gas meter **220** informs the head end system **230** of the gas level and the valve status. Moreover, the gas meter **220** informs the head end system **230** as to the gas level and as to whether the gas valve was turned off based on the detected gas level. The gas meter **220** will periodically exchange data with both the methane sensor **210** and the head end system **230.** The gas meter **220** can receive wakeup notifications whenever the methane sensor **210** detects a gas leak. The gas meter **210** can periodically report to the head end system **230** its detected gas level and its valve status as well.

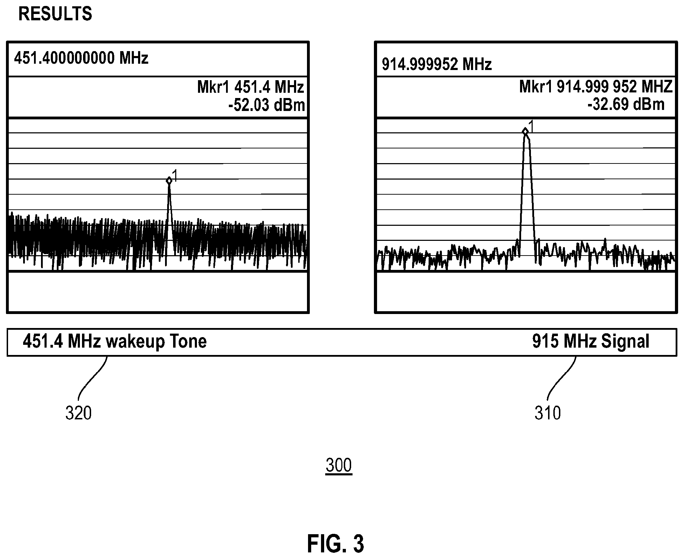

In **FIG. 3****,** a system **300** showing different communication frequencies for the gas meter's communication with the gas detector and head end system is illustrated. When the gas meter is in sleep mode, and the gas detector detects a gas level that could amount to a gas leak, the gas detector will send a wakeup notification to the gas meter at the wakeup frequency **320** of 451.4 Mhz. The gas meter will receive the gas level information from gas detector at frequency 915 Mhz **310** and then determine whether the detected gas level is above or below the threshold level, and thereby determine if the detected gas level amounts to a gas leak. Further, as described above, the gas meter will close the gas valve within the gas meter or leave the gas valve open based on the comparison. In addition, the gas meter will report its data to the head end system.

Referring to **FIG. 3****,** the communication frequency **310** of 915 Mhz is also illustrated. The gas meter will communicate periodically with the gas detector and head end system periodically. The gas meter will notify the head end system of its gas level and valve status using the communication frequency **310.** In addition, the gas meter will communicate with the gas detector at the communication frequency **310** regarding the valve status and detected gas level as well.

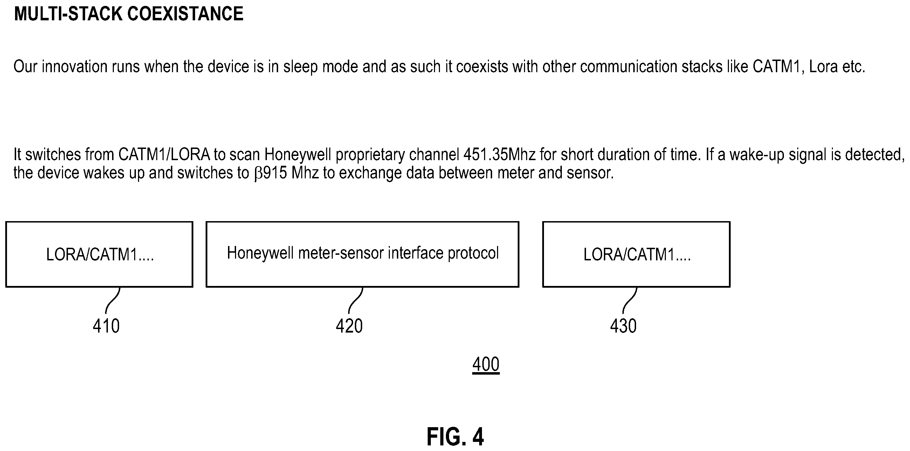

In **FIG. 4****,** a multi-stack coexistence **400** for the gas meter is illustrated. The gas meter can be in sleep mode when the gas detector in the system detects a gas level that could potentially be a gas leak within the gas meter. The gas meter can switch from LORA/CATM1 **410, 430** to scan for the proprietary channel or Honeywell meter-sensor interface protocol **420** at frequency of 451.35 or 451.4 Mhz to determine if a wakeup signal was sent by the gas detector. If the gas meter determines that the gas detector has sent the wake-up signal and detects the wakeup signal, the gas meter will wake up and switch to the 915 Mhz signal to exchange data between the head end system and the gas detector. The gas meter will also send an acknowledgement to the gas detector that the wakeup signal was received. Accordingly, when the gas meter is in sleep mode, the gas meter can scan for the potential wakeup signal. If the gas meter has detected the potential wakeup signal, the gas meter will wakeup and switch to the communication frequency of 915 Mhz to communicate with the gas detector and the head end system.

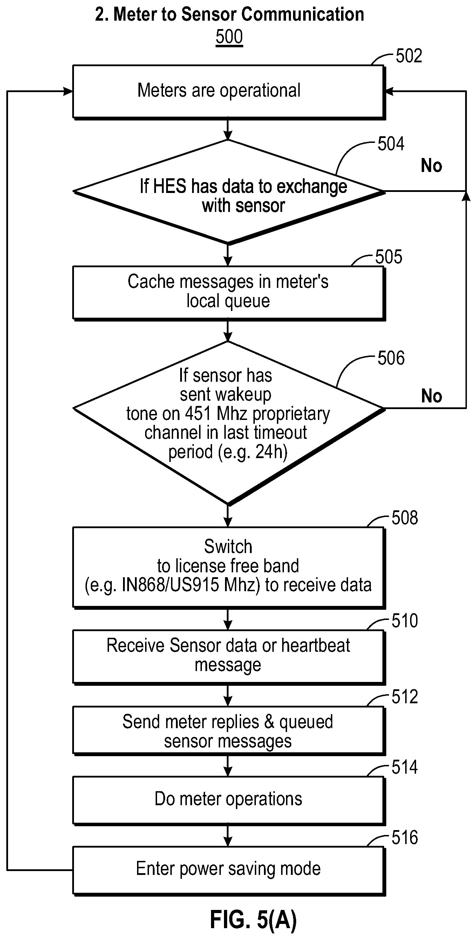

In **FIG. 5(A)****,** meter to sensor communication **500** between the gas meter and the gas detector with a methane sensor, propane sensor, or environmental sensor is illustrated.

In **FIG. 5(A)****,** at step **502** the gas meter remains operational. The gas meter can be in sleep mode while the gas detector can detect a gas level that can amount to a gas leak within the gas meter. At step **504,** a determination is made if the head end system has data to exchange with the methane sensor within the gas detector. If the head end system does not have data to exchange with the gas detector, then the process goes back to step **502.** In contrast, if the head end system has data to exchange with the sensor in the gas detector, then messages are cached in the gas meter's local queue at step **505.** At step **506,** whether the sensor within the gas detector has sent the wakeup tone at 451.4 Mhz on the proprietary channel in the last timeout period to the gas meter is determined when the gas meter is in sleep mode. If the wakeup tone was not sent, the process goes back to step **502.** However, if the wakeup tone was received, then at step **508,** the gas meter switches to a license free band at 915 Mhz to receive data from the gas detector, acknowledges the data reception from gas detector and also communicates with the head end system in 915 Mhz frequency.

Referring to **FIG. 5(A)****,** at step **510,** the gas meter receives the sensor data and or heartbeat message. The sensor data can include the notification on the gas level that could amount to a gas leak within the gas meter, and cause the gas meter to turn off a valve within the gas meter as a result. At step **512,** the gas detector sends the gas meter replies and queued sensor messages. At step **514,** the gas meter performs operations such as comparing the detected gas level to the threshold level, and determining whether to turn off the gas valve based on the comparison. At step **516,** the gas meter enters power saving mode.

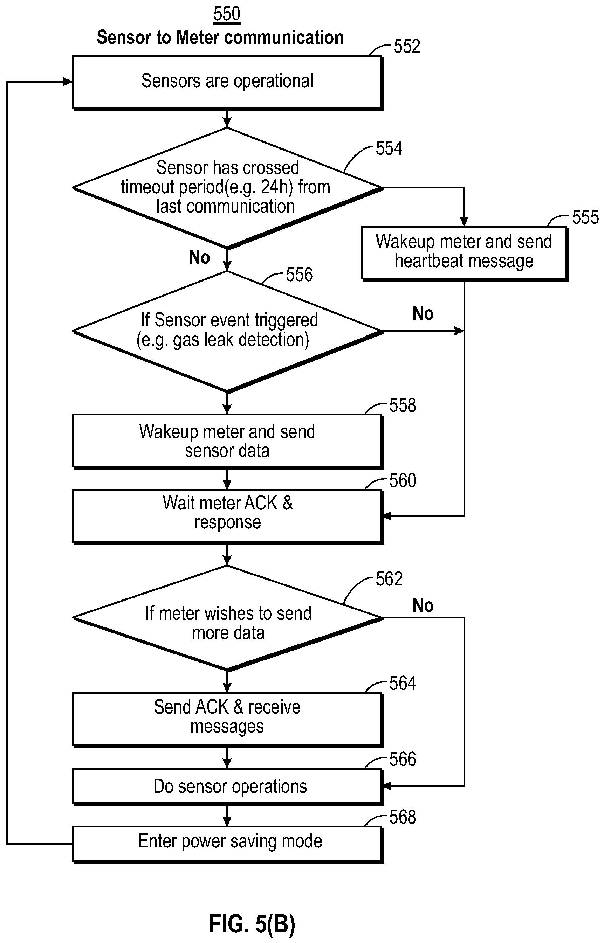

In **FIG. 5(B)****,** the sensor to gas meter communication **550** is illustrated. At step **552,** a determination is made if the sensors (methane, propane, environmental) within the gas detectors are operational. At step **554,** a determination is made as to whether the sensor has crossed the timeout period of twenty-four hours from the last communication with the gas meter. If timeout period has been crossed, then the sensor at step **555** sends the wakeup signal to the gas meter and a heartbeat message is exchanged. If the timeout period has not been crossed, then at step **556,** a check is made on whether a sensor event has occurred, such as a gas leak detection, wherein the sensor has detected a gas level that can be a considered as a gas leak as per its settings. At step **558,** the sensor sends the wakeup signal to the gas meter and sends the sensor data (the gas level) of the detected/suspected gas leak.

Referring to **FIG. 5(B)****,** at step **560,** the sensor waits for the gas meter response and for the gas meter to acknowledge (ACK) that it has received the wakeup signal and gas level information. At step **562,** a determination is made if the gas meter wishes to send more data. If the gas meter does not wish to send more data, then the process moves to step **566.** However, if the gas meter wishes to send more data, the gas meter at step **564** sends an acknowledgement (ACK) and receives messages. At step **566,** the sensor operations are performed such as exchanging data with the gas meter regarding the detected gas level. At step **568,** the sensor enters power saving mode.

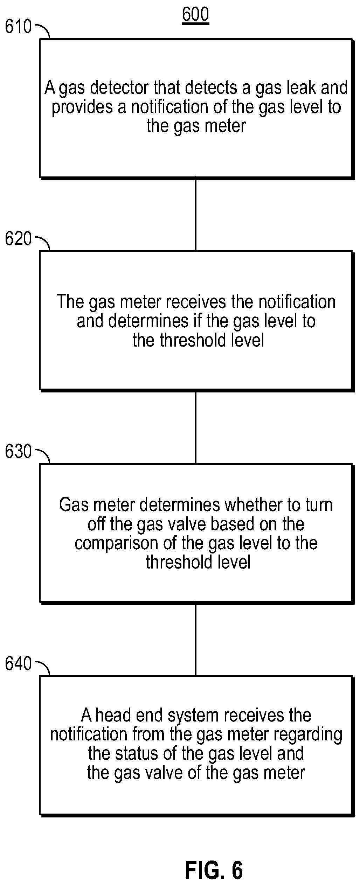

In **FIG. 6****,** a method **600** describing the features of the present invention is described. At step **610,** a gas detector in a system with a gas meter and head end system detects a gas leak and provides a notification of the detected gas level to the gas meter. The gas detector can detect a gas level that indicates a gas leak within the gas meter. The gas meter can be in sleep mode at the time that the gas leak is detected. As a result, the gas detector will send a wakeup notification at the signal of 451.4 Mhz to the gas meter to alert the gas meter when the gas meter is in sleep mode.

In **FIG. 6****,** at step **620,** the gas meter receives the wakeup notification from the gas detector and switches to 915Mhz to receive the gas detector level, and acknowledges to the gas detector that it has received the wakeup notification and the sensor data from the gas detector. The gas meter then communicate with both the gas detector and the head end system. The gas meter also compares the detected gas level to a threshold level.

Referring to **FIG. 6****,** at step **630,** the gas meter determines whether to turn off the gas valve within the gas meter based on the comparison of the gas level to the threshold level. If the gas level is less than the threshold level, the gas meter can decide to leave the gas valve on. However, if the detected gas level is greater than the threshold level, then the gas meter can determine that a gas leak exists and that the gas valve should be turned off.

In **FIG. 6****,** at step **640,** the head end system receives the notification from the gas meter on the detected gas level and the status of the gas valve. The gas meter will report its data to the head end system, and inform the head end system whether a gas leak exists based on the detected gas level, and whether the gas valve was turned off due to the detected gas leak.

Those skilled in the art will appreciate that the example embodiments are non-exhaustive and that embodiments other than that described here may be included without departing from the scope of the presently disclosed embodiments.

### Advantages

Overall, a gas leak can occur when the gas meter is in sleep mode. The gas detector can detect the gas level associated with the gas leak when the gas meter is in sleep mode. As such, the gas detector, or sensor within the gas detector, can send a wakeup signal to the gas detector to notify the gas meter of the suspected gas leak when the gas meter is in sleep mode.

When the gas meter is in sleep mode, the gas meter will receive the wakeup notification from the gas detector regarding the detected gas level. As such, the gas meter is alerted to the potential gas leak due to the wakeup notification from the gas detector. Accordingly, the gas meter can switch to the communication frequency needed to communicate with both the gas detector and the head end system. Further, the gas meter will then compare the detected gas level with a threshold level to confirm if a gas leak exists or not. As such, the gas meter will determine that the gas leak exists if the detected gas level is greater than the threshold level, and, in contrast, determine that no gas leak exists if the detected gas level is less than the threshold level.

The gas meter will also turn off the gas valve when it is determined that the gas leak exists due to the comparison of the detected gas level to the threshold level. The gas meter will also communicate its data to the head end system. The gas meter will inform the head end system of the detected gas level, and gas leak, and whether the gas valve was turned off.

As such, even when in sleep mode, the gas meter can be informed of any potential gas leaks, and receive a wakeup signal by the gas detector to allow the gas meter to determine if a gas leak exits due to the detected gas level. In addition, the gas meter can then turn off the gas valve when the gas meter has determined that the gas leak exits to prevent any further damage from occurring due to the gas leak.

The system supports local pairing/communication between gas meter and gas detector in a way that it can co-exist with other head end system communication stacks such as LoRa/wmBUS/CAT-M1. Thus the gas detector can piggyback on gas meter's HES communication system and avoid additional hardware/mythologies to connect with head-end system independently.

Since the communication model achieved is bi-directional between gas detector and HES (head end system) (via gas meter), the head end system may monitor gas detectors health and other parameters remotely when gas meter is connected to the head system and also update any settings in detector remotely.

In addition, a local pairing/communication model will communicate with the sensor in such a way that gas meter and gas detector communication can co-exist with the gas meter's and head end system communication stack such as LoRa/wmBUS/CAT-M1, etc. the head end system. Thus allowing a gas meter to be built to fully comply with a utility company's specification.

Further, the sensor can piggyback on gas meter's head end system communication model, thereby providing the flexibility for the sensor to avoid another hardware interface model to communicate with. Moreover, wherein a bi-directional communication is established between the sensor and head end system via the gas meter, without disturbing gas meter operations; thus allowing the operator to monitor gas detector's health or other parameters directly and update relevant settings on demand remotely.

The system maintains normal communication with the head end system when gas leak is not detected with a wireless stack of choice and manages to maintain coexistence of said stack with the communication methodology used to communicate with the gas detector, thus having a gas meter which runs normally as per the specification of utility companies. The system also describes a method by which the gas detector could piggy-back on the gas meter's existing infrastructure to establish a direct communication with the head end system and avoid its own hardware/methodology to interface with the head end system.

### Conclusion

All the features disclosed in this specification, including any accompanying abstract and drawings, may be replaced by alternative features serving the same, equivalent or similar purpose, unless expressly stated otherwise. Thus, unless expressly stated otherwise, each feature disclosed is one example only of a generic series of equivalent or similar features.

Various aspects of the invention have been described above by way of illustration, and the specific embodiments disclosed are not intended to limit the invention to the particular forms disclosed. The particular implementation of the system provided thereof may vary depending upon the particular context or application.

## Claims

1. A system comprising:
a gas detector (120) configured to detect a gas leak and provide a notification to a gas meter (110) regarding a gas level of the gas leak;
the gas meter (110) including a gas valve is configured to receive the notification about the gas leak from the gas detector (120), and compare the gas level detected by the gas detector (120) with a threshold level and determine if the detected gas level is greater than the threshold level to determine whether to turn off the gas valve due to the detected gas level; and
a head end system (130) configured to receive notification of the detected gas level and status of the gas valve from the gas meter (110). (110),
**characterized in that** the gas detector (120) sends a wakeup signal to the gas meter (110) and the gas meter is in sleep mode when the gas detector detects the gas level in relation to the gas leak,
and **in that** the gas meter (110) is configured to receive the wakeup signal in one frequency and switches to a different frequency in response to the wake up signal from the gas detector (120) to exchange data with the gas detector.

2. The system of claim 1, wherein the gas meter (110) closes the gas valve when the detected gas level is greater than the threshold level.

3. The system of claim 1, the gas meter (110) periodically reports the status of its gas level and the gas valve to the head end system (130).

4. The system of claim 1, wherein the gas meter (110) determines not to close the gas valve based on the comparison of the detected gas level and the threshold level.

5. The system of claim 1, further comprising:
a methane sensor (210), propane sensor, or environmental sensor configured to send the wakeup signal to the gas meter (220) as the gas meter (220) is in sleep mode.

6. The system of claim 1 wherein:
the gas detector (120) is further configured to at a first position, detect a gas leak of a gas meter (110) and at a second position, sends the wakeup signal to the gas meter (110) due to the gas meter (110) being in sleep mode;
the gas meter (110) is further configured to receive the wakeup signal from the gas detector (120), and after comparing the gas detector level associated with the gas leak to the threshold level, determine whether to close the gas valve within the gas meter (110) based on the comparison, and report its data to the head end system (130); and
wherein the head end system (130) is further configured to receive data that includes the determination as to whether the gas meter (110) turned off the gas valve due to the comparison of the gas level associated with the gas leak to the threshold level.

7. The system of claim 6, wherein the gas meter (110) determines to close the gas valve due to the comparison of the gas level to the threshold level.

8. The system of claim 6, wherein the gas meter (110) reports data regarding the gas level and valve status to the head end system (130) in periodic intervals

## Patentansprüche

1. System, umfassend:
eine Gaserkennungsvorrichtung (120), die dazu konfiguriert ist, ein Gasleck zu erkennen und eine Benachrichtigung an einen Gaszähler (110) in Bezug auf eine Gaskonzentration des Gaslecks bereitzustellen;
wobei der Gaszähler (110) ein Gasventil beinhaltet, das dazu konfiguriert ist, die Benachrichtigung über das Gasleck von der Gaserkennungsvorrichtung (120) zu empfangen und die von der Gaserkennungsvorrichtung (120) erkannte Gaskonzentration mit einer Schwellenwertkonzentration zu vergleichen und zu bestimmen, ob die erkannte Gaskonzentration größer als die Schwellenwertkonzentration ist, um zu bestimmen, ob das Gasventil aufgrund der erkannten Gaskonzentration abzustellen ist;
und
ein Head-End-System (130), das dazu konfiguriert ist, eine Benachrichtigung über die erkannte Gaskonzentration und über einen Status des Gasventils von dem Gaszähler (110) zu empfangen,
**gekennzeichnet dadurch, dass** die Gaserkennungsvorrichtung (120) ein Wecksignal an den Gaszähler (110) sendet und der Gaszähler in einem Schlafmodus ist, wenn die Gaserkennungsvorrichtung die Gaskonzentration im Verhältnis zu dem Gasleck erkennt,
und dadurch, dass der Gaszähler (110) dazu konfiguriert ist, das Wecksignal in einer Frequenz zu empfangen, und als Antwort auf das Wecksignal von der Gaserkennungsvorrichtung (120) auf eine unterschiedliche Frequenz umschaltet, um Daten mit der Gaserkennungsvorrichtung auszutauschen.

2. System nach Anspruch 1, wobei der Gaszähler (110) das Gasventil schließt, wenn die erkannte Gaskonzentration höher ist als die Schwellenwertkonzentration.

3. System nach Anspruch 1, wobei der Gaszähler (110) periodisch den Status seiner Gaskonzentration und des Gasventils an das Head-End-System (130) meldet.

4. System nach Anspruch 1, wobei der Gaszähler (110) bestimmt, auf Basis des Vergleichs der erkannten Gaskonzentration und der Schwellenwertkonzentration das Gasventil nicht zu schließen.

5. System nach Anspruch 1, ferner umfassend:
einen Methansensor (210), Propansensor oder Umgebungssensor, der dazu konfiguriert ist, das Wecksignal an den Gaszähler (220) zu senden, wenn der Gaszähler (220) im Schlafmodus ist.

6. System nach Anspruch 1, wobei:
die Gaserkennungsvorrichtung (120) ferner dazu konfiguriert ist, in einer ersten Position ein Gasleck eines Gaszählers (110) zu erkennen und in einer zweiten Position das Wecksignal an den Gaszähler (110) zu senden, aufgrund dessen, dass der Gaszähler (110) im Schlafmodus ist;
der Gaszähler (110) ferner dazu konfiguriert ist, das Wecksignal von der Gaserkennungsvorrichtung (120) zu empfangen, und nach Vergleichen der dem Gasleck zugeordneten Gasdetektorkonzentration mit der Schwellenwertkonzentration zu bestimmen, ob das Gasventil innerhalb des Gaszählers (110) auf Basis des Vergleichs zu schließen ist, und seine Daten an das Head-End-System (130) zu melden; und
wobei das Head-End-System (130) ferner dazu konfiguriert ist, Daten zu empfangen, die die Bestimmung beinhaltet, ob der Gaszähler (110) aufgrund des Vergleichs der dem Gasleck zugeordneten Gaskonzentration mit der Schwellenwertkonzentration das Gasventil abgeschaltet hat.

7. System nach Anspruch 6, wobei der Gaszähler (110) bestimmt, das Gasventil aufgrund des Vergleichs der Gaskonzentration mit der Schwellenwertkonzentration zu schließen.

8. System nach Anspruch 6, wobei der Gaszähler (110) Daten in Bezug auf die Gaskonzentration und den Ventilstatus in periodischen Intervallen an das Head-End-System (130) meldet.

## Revendications

1. Système comprenant :
un détecteur de gaz (120) configuré pour détecter une fuite de gaz et fournir une notification à un compteur de gaz (110) concernant un niveau de gaz de la fuite de gaz ;
le compteur de gaz (110) incluant une vanne de gaz est configuré pour recevoir la notification concernant la fuite de gaz en provenance du détecteur de gaz (120), et comparer le niveau de gaz détecté par le détecteur de gaz (120) avec un niveau seuil et déterminer si le niveau de gaz détecté est supérieur au niveau seuil pour déterminer s'il faut fermer la vanne de gaz du fait du niveau de gaz détecté ;
et
un système de tête de réseau (130) configuré pour recevoir une notification du niveau de gaz détecté et de l'état de la vanne de gaz en provenance du compteur de gaz (110),
**caractérisé en ce que** le détecteur de gaz (120) envoie un signal d'activation au compteur de gaz (110) et le compteur de gaz est en mode veille lorsque le détecteur de gaz détecte le niveau de gaz par rapport à la fuite de gaz,
et **en ce que** le compteur de gaz (110) est configuré pour recevoir le signal d'activation dans une fréquence et commute vers une fréquence différente en réponse au signal d'activation provenant du détecteur de gaz (120) pour échanger des données avec le détecteur de gaz.

2. Système selon la revendication 1, dans lequel le compteur de gaz (110) ferme la vanne de gaz lorsque le niveau de gaz détecté est supérieur au niveau seuil.

3. Système selon la revendication 1, dans lequel le compteur de gaz (110) signale périodiquement l'état de son niveau de gaz et de la vanne de gaz au système de tête de réseau (130).

4. Système selon la revendication 1, dans lequel le compteur de gaz (110) détermine de ne pas fermer la vanne de gaz sur la base de la comparaison du niveau de gaz détecté et du niveau seuil.

5. Système selon la revendication 1, comprenant en outre :
un capteur de méthane (210), un capteur de propane ou un capteur environnemental configuré pour envoyer le signal d'activation au compteur de gaz (220) lorsque le compteur de gaz (220) est en mode veille.

6. Système selon la revendication 1 dans lequel :
le détecteur de gaz (120) est en outre configuré, dans une première position, pour détecter une fuite de gaz d'un compteur de gaz (110) et dans une seconde position, pour envoyer le signal d'activation au compteur de gaz (110) du fait que le compteur de gaz (110) est en mode veille ;
le compteur de gaz (110) est en outre configuré pour recevoir le signal d'activation en provenance du détecteur de gaz (120) et, après avoir comparé le niveau de détecteur de gaz associé à la fuite de gaz au niveau seuil, déterminer s'il faut fermer la vanne de gaz à l'intérieur du compteur de gaz (110) sur la base de la comparaison, et transmettre ses données au système de tête de réseau (130) ; et
dans lequel le système de tête de réseau (130) est en outre configuré pour recevoir des données qui incluent le fait de déterminer si le compteur de gaz (110) a fermé la vanne de gaz du fait de la comparaison du niveau de gaz associé avec la fuite de gaz au niveau seuil.

7. Système selon la revendication 6, dans lequel le compteur de gaz (110) détermine de fermer la vanne de gaz du fait de la comparaison du niveau de gaz et du niveau seuil.

8. Système selon la revendication 6, dans lequel le compteur de gaz (110) transmet des données concernant le niveau de gaz et l'état de la vanne au système de tête de réseau (130) à intervalles périodiques.
